# FASCICULE DE BREVET EUROPEEN

(11) **EP 1 682 207 B1**
(45) Date de publication et mention de la délivrance du brevet: **18.07.2012**
(21) Numéro de dépôt: 04817285.2
(22) Date de dépôt: 18.10.2004
(51) Int. Cl.: A61M 5/50, A61M 5/315, A61M 5/32

(54) **DISPOSITIF DE SERINGUE D'INJECTION SECURISE**
GESCHÜTZTE INJEKTIONSSPRITZENVORRICHTUNG
PROTECTED INJECTION SYRINGE DEVICE

(30) Priorité: 22.10.2003 FR 0312327
(43) Date de publication de la demande: 26.07.2006
(73) Titulaire: Tech Group Europe Limited, Dublin 2 (IE)
(72) Inventeur: CHEVALLIER, Stéphane, F-77178 Saint Pathus (FR)
(74) Mandataire: Intès, Didier Gérard André
(86) Numéro de dépôt international: PCT/FR2004/002654
(87) Numéro de publication internationale: WO 2005/039678

(56) Documents cités:
- WO-A-98/35714
- WO-A-02/072182
- FR-A- 2 653 667
- FR-A- 2 835 753
- US-B1- 6 319 234

## Description

La présente invention concerne un dispositif d'injection sécurisé, comprenant une seringue ayant un corps de seringue, une aiguille et un piston apte à être déplacé dans ce corps pour une injection, et des moyens de sécurité qui comportent un fourreau de protection, le corps de seringue et le fourreau de protection étant susceptibles de coulisser l'un par rapport à l'autre entre une configuration d'injection dans laquelle l'aiguille dépasse hors du fourreau de protection qui est disposé autour du corps de seringue, et une configuration de protection dans laquelle l'aiguille s'étend à l'intérieur dudit fourreau, le dispositif comprenant un organe de déclenchement apte à provoquer le passage de la configuration d'injection à la configuration de protection en fin de course d'injection.

Des dispositifs de ce type sont connus, par exemple par les documents FR 2 801 795, EP 0 966 983, ou encore EP 0 740 942.

Pour l'injection du produit que contient le corps de seringue dans le corps d'un patient, ce corps de seringue et le fourreau de protection sont dans leur configuration d'injection. Après cette injection, l'organe de déclenchement permet de les faire passer dans la configuration de protection pour éviter que l'utilisateur ne se pique accidentellement avec l'aiguille et limiter par conséquent les risques de contamination éventuelle.

La société demanderesse a constaté qu'il existe des situations dans lesquelles il est souhaitable d'éviter que, en fin de course d'injection du piston, le dispositif n'adopte sa configuration de protection. C'est par exemple le cas lorsqu'un tel dispositif est utilisé avec une seringue non pré-remplie, devant être manipulée avant l'injection. En effet, pour remplir une telle seringue, il est nécessaire que le piston soit placé en fin de course vers l'avant et, à partir de cette situation, de déplacer le piston vers l'arrière, c'est-à-dire dans le sens allant en s'éloignant de l'aiguille, pour que le liquide à injecter pénètre dans le corps de seringue. Il convient alors que, lors de ce remplissage et lors de l'injection qui va suivre, le corps de seringue et le fourreau de protection soient dans leur configuration d'injection.

WO 98/35714 divulgue un dispositif conforme au préambule de la revendication 1.

La présente invention a pour but de proposer un dispositif d'injection sécurisé de type précité dans lequel le passage en configuration de protection est déclenché en fin de course d'injection, mais de manière non systématique.

Ce but est atteint grâce aux caractéristiques de la revendication 1.

Lors des éventuelles manipulations de la seringue avant l'injection, c'est la première situation de fin de course d'injection qui est définie de manière à empêcher un passage intempestif en configuration de protection, tandis que c'est la deuxième situation de fin de course d'injection qui est définie lors de l'injection.

Avantageusement, le dispositif comprend un organe inhibiteur apte à occuper une position d'inhibition dans laquelle la situation de fin de course d'injection est ladite première situation et à être déplacé par rapport à cette position d'inhibition pour permettre que la situation de fin de course d'injection soit ladite deuxième situation.

Cet organe inhibiteur constitue de préférence une pièce aisément manipulable qui, initialement, est dans sa position d'inhibition.

Selon un premier mode de réalisation avantageux, l'organe de déclenchement est solidaire du déplacement du piston et le dispositif comprend des moyens pour définir une première et une deuxième position de fin de course d'injection du piston correspondant respectivement aux première et deuxième situations de fin de course d'injection.

De préférence, on fait en sorte que les deux positions de fin de course d'injection du piston soient proches l'une de l'autre, afin que, dans les deux cas, le corps de seringue ne contienne pas ou pratiquement pas d'air ou de liquide.

Dans ce cas, avantageusement, le dispositif comprend des moyens de butée aptes à être mis en service pour définir la première position de fin de course d'injection et hors service pour permettre l'obtention de la deuxième position de fin de course d'injection.

Lorsque ces moyens de butée sont en service, ils définissent la première position de fin de course d'injection pour le piston, dans laquelle l'extrémité avant de ce piston est située au voisinage de l'extrémité avant du corps de seringue à laquelle est disposée l'aiguille, sans toutefois atteindre cette extrémité avant. En revanche, lorsque les moyens de butée sont hors service, l'extrémité avant du piston peut aller légèrement au-delà de cette première position de fin de course d'injection.

Avantageusement, dans sa position d'inhibition, l'organe inhibiteur est raccordé au piston en étant solidaire du déplacement de ce dernier et est apte à coopérer en butée avec un élément du dispositif fixe par rapport au corps de seringue pour définir la première position de fin de course d'injection.

L'organe inhibiteur étant initialement dans sa position d'inhibition, la première position de fin de course d'injection est automatiquement obtenue en poussant le piston vers l'avant jusqu'à ce que l'organe inhibiteur vienne en butée. Pour permettre l'obtention de la deuxième position de fin de course d'injection, il suffit de déplacer l'organe inhibiteur, soit en le déplacement vers l'arrière par rapport au piston, soit par exemple en le séparant de ce piston pour que, à l'issue de l'injection dans le corps d'un patient, le piston puisse parvenir dans sa deuxième position de fin de course d'injection.

Selon un deuxième mode de réalisation avantageux, l'organe de déclenchement est raccordé au piston et est apte à être déplacé par rapport à ce dernier entre une position apte au déclenchement dans laquelle, en fin de course d'injection du piston, ledit organe de déclenchement est apte à provoquer le passage de la configuration d'injection à la configuration de protection et une position inapte au déclenchement dans laquelle, en fin de course d'injection du piston, l'organe de déclenchement est inapte à provoquer le passage de la configuration d'injection à la configuration de protection.

Dans ce cas, l'organe de déclenchement est placé dans sa position inapte au déclenchement pour les manipulations du dispositif préalables à une injection. Lorsque ces manipulations sont terminées, la seringue étant alors prête pour injection, l'organe de déclenchement peut être placé dans sa position apte au déclenchement afin que la configuration de protection soit obtenue à la fin de l'injection.

Dans ce cas, avantageusement, l'organe de déclenchement est mobile axialement par rapport au piston, la position apte au déclenchement étant décalée vers l'extrémité du piston dirigée vers l'aiguille par rapport à la position inapte au déclenchement.

L'invention sera bien comprise et ses avantages apparaîtront mieux à la lecture de la description détaillée qui suit, de modes de réalisation présentés à titre d'exemples non limitatifs. La description se réfère aux dessins annexés sur lesquels :
- la figure 1 est une vue d'un dispositif conforme à l'invention, en coupe longitudinale, sur laquelle le corps de seringue et le fourreau de protection sont dans leur configuration d'injection ;
- la figure 2 est une vue partielle du dispositif de la figure 1, en coupe selon la ligne II-II de la figure 1 ;
- la figure 3 est une vue partielle du dispositif, en perspective vue de l'arrière, selon la flèche III de la figure 1;
- les figures 4 et 5 sont des vues analogues aux figures 1 et 2, montrant la première situation de fin de course d'injection, la figure 5 étant une coupe partielle ;
- la figure 6 est une vue dans le même plan de coupe que les figures 2 et 5, montrant la deuxième situation de fin de course d'injection et la configuration de protection ;
- la figure 7 est une vue analogue à la figure 4, pour un deuxième mode de réalisation ; et
- la figure 8 est une vue analogue à la figure 6, pour ce deuxième mode de réalisation.

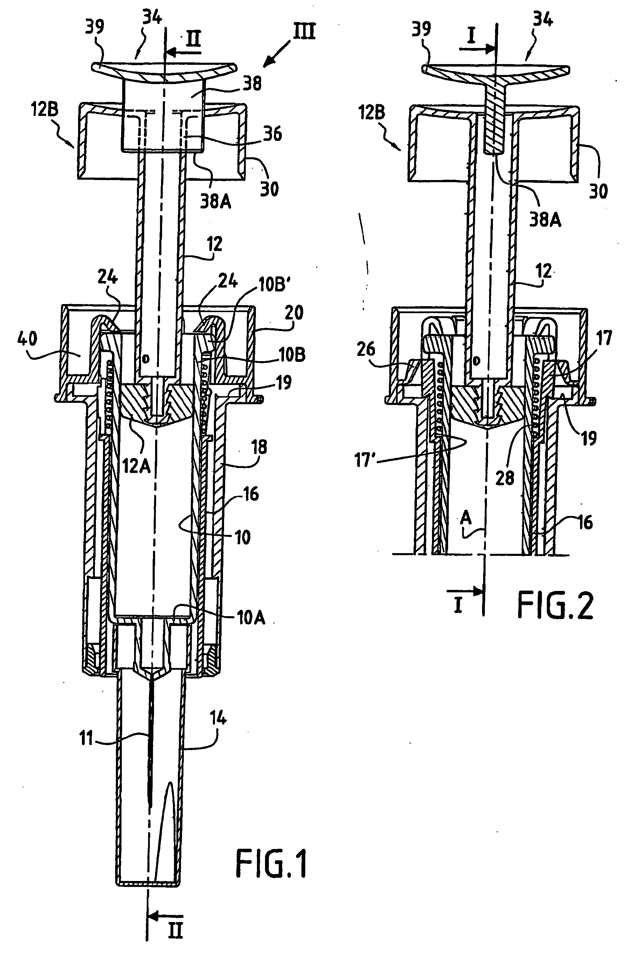

Le dispositif représenté sur les figures comprend une seringue qui a un corps de seringue 10, une aiguille 11 et un piston 12 qui peut coulisser dans ce corps pour une injection. Sur les figures 1 et 2, le piston 12 est dans sa position d'attente d'injection, dans laquelle son extrémité avant 12A est située vers l'extrémité arrière 10B, éloignée de l'aiguille, du corps de seringue 10.

Le dispositif est représenté avant l'injection et, de manière connue en soi, l'aiguille 11 est protégée par un capuchon de protection 14 qui est rapporté à l'extrémité avant 10A du corps de seringue 10.

Le dispositif comporte également des moyens de sécurité qui comprennent un fourreau de protection 16.

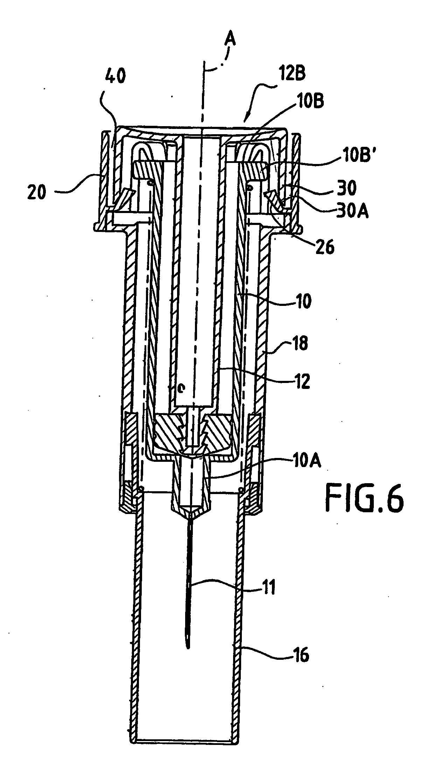

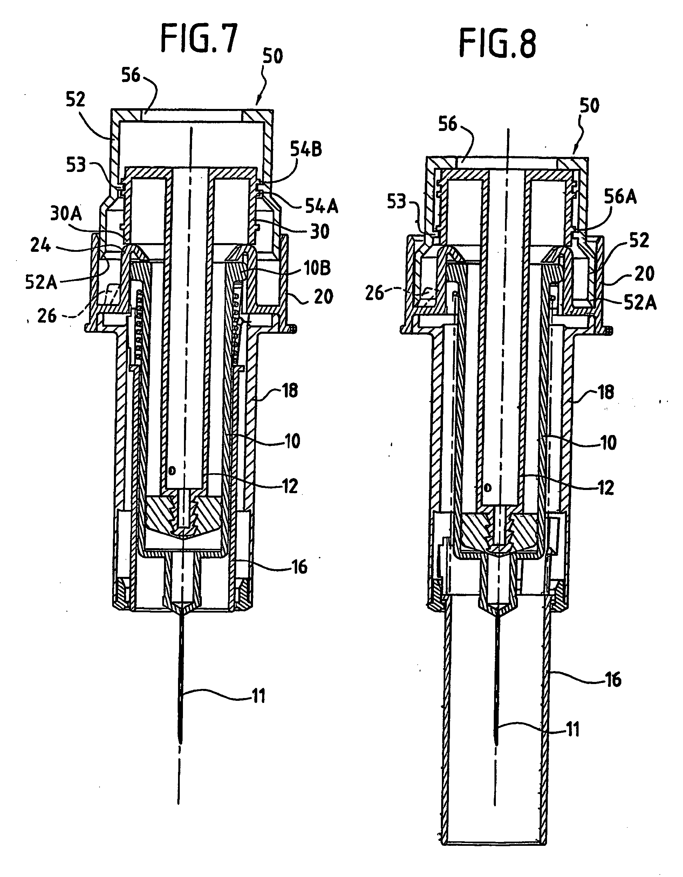

Sur les figures 1 à 5 et 7, ce fourreau de protection 16 est dans une position d'attente, dans laquelle il est disposé autour du corps de seringue 10 ; c'est la configuration d'injection. Sur les figures 6 et 8, le déplacement de ce fourreau par rapport au corps de seringue 10 a été déclenché, et l'on voit que le fourreau 16 dépasse au-delà de l'extrémité avant 10A du corps de seringue de telle sorte que l'aiguille 11 s'étend à l'intérieur du fourreau ; c'est la configuration de protection.

Dans l'exemple représenté, les moyens de protection sont analogues à ceux que divulgue le document FR 2 801 795 et, en fin d'injection, c'est le fourreau 16 qui se déplace par rapport au corps de seringue pour avancer et venir se placer autour de l'aiguille.

Plus précisément, le corps de seringue est disposé dans un fourreau de support 18 à l'intérieur duquel le fourreau de protection 16 est escamoté en position d'attente, en étant disposé dans un espace annulaire ménagé entre le corps de seringue et ce fourreau de support.

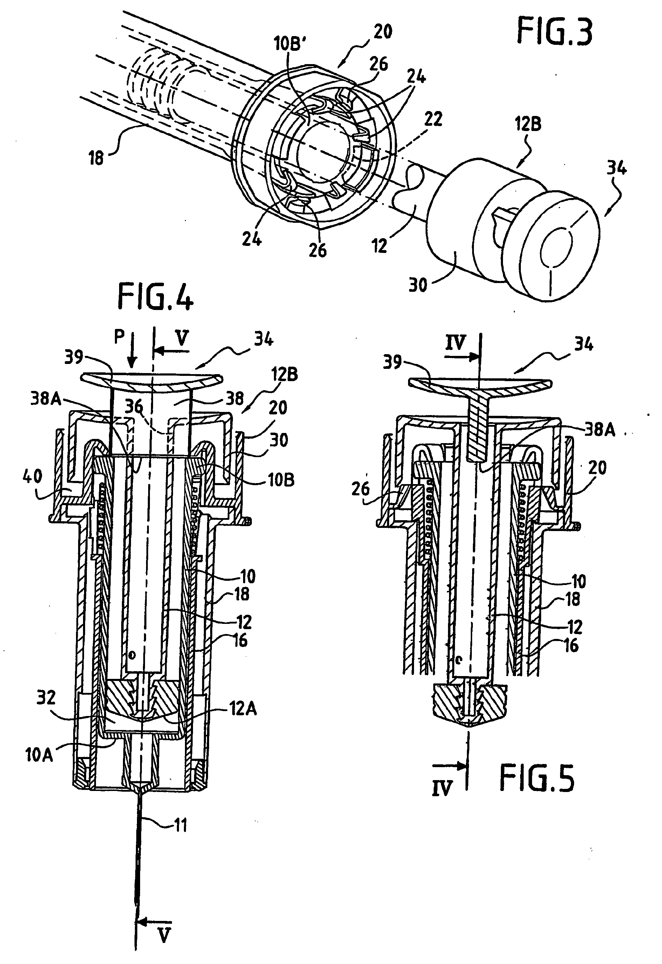

L'extrémité arrière du fourreau de support 18 est formée par une partie d'extrémité 20 qui permet de retenir le corps de seringue de manière fixe à l'intérieur du fourreau de support par la collerette 10B' de cette extrémité arrière. Plus précisément, comme on le voit sur la figure 3, la partie 20 présente des éléments de paroi radiale 22 formant des portions d'épaulement tournées vers l'arrière sur lesquelles repose la collerette 10B' de manière à empêcher que le corps de seringue se déplace vers l'avant par rapport au fourreau de support. La partie 20 comporte également des languettes de retenue 24 qui sont élastiques et s'écartent radialement lors de l'insertion de la collerette 10B' dans la partie 20 jusqu'aux portions d'épaulement 22, avant de reprendre leur position initiale pour retenir cette collerette vis-à-vis d'un déplacement vers l'arrière.

Par ailleurs, l'extrémité arrière du fourreau 18 présente, dans sa zone de raccordement avec la partie 20, un épaulement intérieur 19 tourné vers l'arrière sur lequel, dans la configuration d'injection, des pattes élastiques 17 de l'extrémité arrière du fourreau de protection 16 viennent s'accrocher pour empêcher le déplacement de ce fourreau de protection vers l'avant.

La partie 20 présente des pattes d'actionnement 26 qui, comme on le voit sur la figure 2, sont situées en regard des pattes 17.

Au voisinage de son extrémité arrière, le fourreau de protection 16 présente un décrochement 17' formant un épaulement intérieur tourné vers l'arrière pour l'extrémité avant d'un ressort 28. L'extrémité arrière de ce ressort repose contre la face avant des éléments de paroi qui constituent les portions d'épaulement 22 précédemment évoquées. Ainsi, en configuration d'injection, le ressort 28 est comprimé.

La tête arrière 12B du piston 12 présente une jupe 30 qui constitue un organe de déclenchement pour faire passer le dispositif dans sa configuration de protection.

En effet, comme on le voit sur la figure 6, lorsque le piston 12 est avancé au maximum à l'intérieur du corps de seringue 10, l'extrémité avant 30A de cette jupe coopère avec les pattes élastiques 26 pour repousser celles-ci radialement vers l'intérieur, c'est-à-dire vers l'axe A du dispositif, de telle sorte que ces pattes repoussent les pattes 17 du fourreau de protection également vers l'intérieur. Alors, ce dernier n'est plus retenu par l'épaulement 19 et peut s'avancer sous l'effet de l'effort exercé par le ressort 28 jusqu'à atteindre la position représentée à la figure 6 dans laquelle le dispositif est dans sa configuration de protection.

Les moyens de sécurité qui viennent d'être décrits correspondent à ceux que défini le document FR 2 801 795 et le corps de seringue est fixe, tandis que c'est le fourreau de protection qui avance par rapport à lui pour obtenir la configuration de protection. Bien entendu, l'invention s'applique également à des dispositifs inverses, dans lesquels le corps de seringue recule par rapport au fourreau de protection pour définir la configuration de protection.

Dans les deux cas, cette configuration de protection est obtenue en fin de course d'injection.

Le dispositif de l'invention comprend des moyens pour définir deux situations distinctes de fin de course d'injection, l'une dans laquelle le déclenchement de la configuration d'injection n'est pas réalisé, et l'autre dans laquelle ce déclenchement est réalisé.

Dans l'exemple des figures 1 à 6, l'organe de déclenchement constitué par la jupe 30 est solidaire du déplacement du piston 12 et les première et deuxième situations de fin de course d'injection correspondent respectivement à une première et à une deuxième position de fin de course d'injection du piston.

La première position de fin de course d'injection est représentée sur les figures 4 et 5 et l'on voit que l'extrémité avant 12A du piston 12 ne parvient pas tout à fait au contact de l'extrémité avant 10A du corps de seringue. L'espace 32 qui est délimité entre ces deux extrémités est exagéré sur le dessin pour la parfaite compréhension de l'invention. Toutefois, cet espace a un très faible volume et on peut même prévoir que, dans cette première position de fin de course, l'extrémité avant du piston vienne au contact de la paroi formée à l'extrémité avant 10A du corps de seringue, car cette extrémité avant du piston E est formée par une pièce souple, par exemple en élastomère, dont la déformabilité lui permet d'être légèrement comprimée pour, dans la deuxième configuration de fin de course, permettre que le piston avance davantage vers l'avant pour occuper sa deuxième position de fin de course comme le montre la figure 6.

Dans le premier mode de réalisation, le dispositif comprend un organe inhibiteur 34 qui, sur les figures 1 à 5, est représenté dans sa position d'inhibition dans laquelle il est raccordé à la tête 12B du piston 12. Dans l'exemple représenté, l'organe inhibiteur 34 est formé par une pièce de tête 34 qui est rapportée sur la tête 12B du piston dans sa positon d'inhibition et qui peut être séparée de cette tête pour permettre l'obtention de la deuxième position de fin de course d'injection du piston. Plus précisément, dans sa position d'inhibition représentée sur les figures 1 à 5, l'organe inhibiteur 34 traverse la tête du piston qui, à cet effet, présente une fente 36. C'est une portion longitudinale de la pièce 34, formée par un tenon ou par un élément de paroi 38, qui passe à travers cette fente. L'extrémité avant 38A de ce tenon ou de cet élément de paroi se trouve donc dans un espace intérieur de la tête du piston ménagé entre la face interne de la jupe 30 et la tige du piston. Dans la position d'inhibition de la pièce 34, le tenon 38 avance vers l'extrémité du piston opposée à sa tête 12B, plus loin que la jupe 30.

Comme on le voit sur la figure 4, l'extrémité avant 38A de l'élément de paroi 38 de l'organe inhibiteur 34, vient en butée contre l'extrémité arrière 10B du corps de seringue 10 pour définir la première position de fin de course d'injection. En effet, pour déplacer le piston vers l'avant, l'utilisateur appuie naturellement sur l'organe inhibiteur 34 ou, plus précisément, sur la tête d'actionnement 39 de cet organe. On comprend que, dans la position de la figure 4, une poussée P sur cette tête 39 n'a plus aucun effet.

Il convient de relever que, pour définir la première position de fin de course d'injection, l'organe inhibiteur pourrait coopérer en butée avec un autre élément du dispositif que l'extrémité arrière du corps de seringue. Il peut en effet s'agir de tout élément fixe par rapport au corps de seringue, en particulier de toute partie appropriée du fourreau extérieur 18 ou de la partie 20.

Dans le mode de réalisation des figures 1 à 6, il suffit, pour permettre l'obtention de la deuxième position de fin de course, de séparer l'organe inhibiteur 34 de la tête du piston. C'est ce que montre la figure 6, sur laquelle l'organe inhibiteur est enlevé, permettant ainsi que la jupe 30 parvienne jusqu'au contact des pattes d'actionnement 26 ce qui n'était pas possible sur les figures précédentes.

Sans sortir du cadre de l'invention, on pourrait modifier la conformation de la tête du piston et de l'organe inhibiteur pour faire en sorte que, à partir de sa position d'inhibition, celui-ci puisse être déplacé vers l'arrière par rapport à la tête de telle sorte que son extrémité avant 38A soit reculée afin qu'elle en vienne plus en butée contre l'extrémité arrière du corps de seringue en fin d'injection, permettant ainsi l'obtention de la deuxième position de fin de course d'injection. Pour retenir l'organe inhibiteur dans cette position reculée, on pourrait par exemple choisir qu'il coopère avec la tête du piston par un système à baïonnette ou équivalent.

Dans l'exemple représenté, l'espace intérieur de la partie 20 forme un logement 40 dans lequel, comme on le voit sur la figure 6, la tête 12B du piston est sensiblement escamotée dans la deuxième position de fin de course d'injection, de sorte que le piston n'est plus ou pratiquement plus utilisable. On constate en revanche sur les figures 4 et 5 que, dans la première position de fin de course d'injection, la tête du piston dépasse hors de ce logement. Elle offre ainsi à l'utilisateur une prise qui permet de tirer le piston vers l'arrière, pour préparer le dispositif à une injection.

On décrit maintenant les figures 7 et 8, qui montrent un deuxième mode de réalisation pour les moyens qui permettent d'obtenir les première et deuxième situations de fin de course d'injection. Sur ces figures, l'organe de déclenchement est formé par la jupe 52 d'une coiffe 50 qui est disposée autour de la tête 12B du piston et qui est déplaçable par rapport à cette dernière.

En effet, la figure 7 montre la coiffe 50 dans sa position inapte au déclenchement, dans laquelle elle est reculée vers l'arrière par rapport à la tête 12B. Ainsi, l'extrémité avant 52A de la jupe 52 ne parvient pas jusqu'au contact des pattes d'actionnement 26 précédemment évoquées en fin de course d'injection. Pour la clarté de l'explication, une patte d'actionnement 26 est représentée en traits mixtes sur les figures 7 et 8 bien qu'elle ne soit pas dans le plan de coupe de ces figures.

En revanche, sur la figure 8, la coiffe 50 est avancée sur la tête 12B du piston de telle sorte que sa jupe 52 puisse, en fin de course d'injection, coopérer avec les languettes 26 par son extrémité avant comme le fait la jupe 30 du mode de réalisation précédent sur la figure 6.

Avantageusement, le coulissement de l'organe de déclenchement 50 par rapport au piston 12 comporte un point dur pour maintenir cet organe de déclenchement dans sa position inapte au déclenchement. En l'espèce, la paroi interne de la jupe 52 présente une nervure de clipsage 53 qui, dans la position inapte au déclenchement de la figure 7, est retenue entre deux nervures 54A et 54B formées sur la paroi externe de la jupe 30 de la tête 12B. Il convient de relever que pour permettre l'actionnement du piston sans déplacer vers l'avant la coiffe 50, la paroi d'extrémité arrière de cette dernière présente une ouverture centrale 56 à travers laquelle l'utilisateur peut introduire un doigt afin d'appuyer directement sur la tête 12B du piston.

A partir de la situation de la figure 7, le piston peut être ramené vers l'arrière en le manipulant par la coiffe 50, qui est retenue par la nervure 54B vis-à-vis d'un déplacement vers l'arrière.

Lorsque la tête du piston est reculée de manière à être écartée du fourreau de support 18, l'utilisateur peut retenir la tige de piston entre deux doigts en poussant vers l'avant la pièce 50 avec un autre doigt de manière à dépasser le point dur précité. En l'espèce, la paroi externe de la jupe 30 présente une autre nervure 56A, qui est située au voisinage de son extrémité avant et devant laquelle est disposée la nervure 53 de la jupe 52 lorsque la coiffe 50 est dans sa position apte au déclenchement.

On voit que, dans la position de la coiffe 50 inapte au déclenchement, c'est l'extrémité avant 30A de la jupe 30 de la tête du piston qui empêche que ce piston ne soit déplacé vers l'avant au-delà de sa première position de fin de course représentée à la figure 7, en coopérant en butée avec un élément fixe par rapport au corps de seringue, en l'espèce constitué par les extrémités arrières des languettes 24.

Dans les exemples représentés, les moyens (organe inhibiteur 54 ou organe de déclenchement 50) qui, associés au piston, permettent d'éviter un passage systématique en configuration de protection en fin d'injection sont rapportés sur la tête du piston et peuvent en être séparés ou bien être déplacés par rapport à cette tête pour permettre l'obtention de la deuxième situation de fin de course d'injection. Il s'agit dans ces deux exemples de moyens externes au piston. Bien entendu, on pourrait utiliser un organe interne au piston, par exemple passant par l'intérieur de la tige creuse de ce dernier pour pouvoir coopérer ou non en butée avec un élément fixe du dispositif, par exemple la collerette de la seringue ou un élément solidaire de la partie 20.

## Revendications

1. Dispositif d'injection sécurisé, comprenant une seringue ayant un corps de seringue (10), une aiguille (11) et un piston (12) apte à être déplacé dans ce corps pour une injection, et des moyens de sécurité qui comportent un fourreau de protection (16), le corps de seringue et le fourreau de protection (16) étant susceptibles de coulisser l'un par rapport à l'autre entre une configuration d'injection dans laquelle l'aiguille (11) dépasse hors du fourreau de protection (16) qui est disposé autour du corps de seringue (10), et une configuration de protection dans laquelle l'aiguille s'étend à l'intérieur dudit fourreau, le dispositif comprenant un organe de déclenchement (30, 52), solidaire de la tête d'actionnement (12B) du piston (12) et apte à provoquer le passage de la configuration d'injection à la configuration de protection en fin de course d'injection, le dispositif comprenant un organe inhibiteur (34, 50) apte à occuper une position initiale d'inhibition dans laquelle ledit organe inhibiteur (34, 50) définit une première position de fin de course d'injection du piston dans laquelle l'organe de déclenchement (30, 52) est inapte à provoquer le passage de la configuration d'injection à la configuration de protection et à être séparé du piston (12) ou déplacé par rapport à ce dernier pour permettre une deuxième position de fin de course d'injection du piston dans laquelle l'organe de déclenchement (30, 52) est apte à provoquer le passage de la configuration d'injection à la configuration de protection,
**caractérisé en ce que**, dans sa position initiale d'inhibition, l'organe inhibiteur (34, 50) est rapporté sur la tête d'actionnement (12B) du piston (12) de sorte que l'utilisateur appuie sur l'organe inhibiteur (34, 50) pour déplacer le piston (12) jusqu'à sa première position de fin de course d'injection et **en ce que**, pour le passage de la position d'inhibition à ladite deuxième position, l'organe inhibiteur (34, 50) étant déplacé ou séparé par rapport au piston (12), l'utilisateur appuie sur la tête d'actionnement (12B) du piston.

2. Dispositif selon la revendication 1, **caractérisé en ce que**, dans sa position d'inhibition, l'organe inhibiteur (34) traverse la tête (12B) du piston (12).

3. Dispositif selon la revendication 2, **caractérisé en ce qu'**une portion longitudinale de l'organe inhibiteur (34), formée par un tenon ou par un élément de paroi (38), traverse la tête d'actionnement (12B) du piston par une fente (36).

4. Dispositif selon l'une quelconque des revendications 1 à 3, **caractérisé en ce qu'**il comprend des moyens de butée (38A, 10B) aptes à être mis en service pour définir la première position de fin de course d'injection et hors service pour permettre l'obtention de la deuxième position de fin de course d'injection.

5. Dispositif selon la revendication 4, **caractérisé en ce que**, dans sa position d'inhibition, l'organe inhibiteur (34) est apte à coopérer en butée avec un élément (10B) du dispositif fixe par rapport au corps de seringue (10) pour définir la première position de fin de course d'injection.

6. Dispositif selon l'une quelconque des revendications 1 à 5, **caractérisé en ce qu'**il comprend un logement (40) dans lequel la tête (12B) du piston (12) est sensiblement escamotée dans la deuxième position de fin de course d'injection et **en ce que**, dans la première position de fin de course d'injection, la tête du piston dépasse hors de ce logement pour offrir une prise permettant de tirer le piston en éloignant ce dernier de l'aiguille.

7. Dispositif selon la revendication 1, **caractérisé en ce que** l'organe inhibiteur est une coiffe (50), dont une partie de jupe (52) forme l'organe de déclenchement et qui est mobile axialement par rapport au piston (12), la position apte au déclenchement étant décalée vers l'extrémité (12A) du piston (12) dirigée vers l'aiguille par rapport à la position inapte au déclenchement.

8. Dispositif selon la revendication 7, **caractérisé en ce que** l'organe inhibiteur comprend des moyens (53, 54A, 54B) pour définir un point dur dans le coulissement de la coiffe (50) par rapport au piston (12) pour maintenir ledit organe de déclenchement (52) dans sa position inapte au déclenchement.

9. Dispositif selon l'une quelconque des revendications 1 à 8, **caractérisé en ce que** l'organe de déclenchement (30, 52) est formé par une jupe apte à coopérer, via des pattes (26), avec des pattes élastiques (17) du fourreau de protection (16) pour provoquer le passage de la configuration d'injection à la configuration de protection.

10. Dispositif selon l'une quelconque des revendications 1 à 9, **caractérisé en ce que** le fourreau de protection (16) est disposé entre le corps de seringue (10) et un fourreau de support (18).

## Claims

1. A safe injection device comprising a syringe having a syringe body (10), a needle (11), and a piston (12) suitable for moving in the body to perform an injection, and safety means comprising a protective sheath (16), the syringe body and the protective sheath (16) being suitable for sliding relative to each other between an injection configuration in which the needle (11) projects beyond the protective sheath (16) which is disposed around the syringe body (10), and a protection configuration in which the needle extends inside said sheath, the device including a trigger member (30, 52) secured to the actuator head (12B) of the piston (12) and suitable for causing the device to pass from the injection configuration to the protection configuration at the end of the injection stroke, the device comprising an inhibitor member (34, 50) suitable for occupying an initial inhibit position in which said inhibitor member (34, 50) defines a first end-of-injection-stroke position for the piston in which the trigger member (30, 52) is unsuitable for causing the device to pass from the injection configuration to the protection configuration, and for being separated from the piston (12) or displaced relative thereto to enable the piston to reach a second end-of-injection-stroke position in which the trigger member (30, 52) is suitable for causing the device to pass from the injection configuration to the protection configuration,
**characterized in that** in its initial inhibit position, the inhibitor member (34, 50) is supported on the actuator head (12B) of the piston (12) so that the user presses on the inhibitor member (34, 50) to move the piston up to its first end-of-injection-stroke position and **in that**, for passing from the inhibit position to said second position, the inhibitor member (34, 50) being displaced relative to the piston (12) or separated therefrom, the user presses on the actuator head (12B) of the piston.

2. A device according to claim 1, **characterized in that**, in its inhibit position, the inhibitor member (34) passes through the head (12B) of the piston (12).

3. A device according to claim 2, **characterized in that** a longitudinal portion of the inhibitor member (34), formed by a tenon or by a wall element (38), passes through the actuator head (12B) of the piston via a slot (36).

4. A device according to any one of claims 1 to 3, **characterized in that** it includes abutment means (38A, 10B) suitable for being put into operation to define the first end-of-injection-stroke position and for being taken out of operation to enable the second end-of-injection-stroke position to be reached.

5. A device according to claim 4, **characterized in that**, in its inhibit position, the inhibitor member (34) is suitable for co-operating in abutment with an element (10B) of the device that is stationary relative to the syringe body (10) in order to define the first end-of-injection-stroke position.

6. A device according to any one of claims 1 to 5, **characterized in that** it comprises a housing (40) in which the head (12B) of the piston (12) is substantially retracted in the second end-of-injection-stroke position and **in that**, in the end-of-injection-stroke position, the head of the piston projects beyond this housing to offer a purchase enabling the piston to be pulled away from the needle.

7. A device according to claim 1, **characterized in that** the inhibitor member is a cover (50) of which a skirt portion (52) forms the trigger member (50) and which is axially movable relative to the piston (12), the position suitable for triggering being offset towards the end (12A) of the piston (12) that is directed towards the needle relative to the position that is unsuitable for triggering.

8. A device according to claim 7, **characterized in that** the inhibitor member comprises means (53, 54A, 54B) for defining a hard point in the sliding movement of the cover (50) relative to the piston (12) for holding said trigger member in its position that is unsuitable for triggering.

9. A device according to any one of claims 1 to 8, **characterized in that** the trigger member (30, 52) is formed by a skirt suitable for cooperating, via tabs (26), with elastic tabs (17) of the protective sheath (16) for causing the device to pass from the injection configuration to the protection configuration.

10. A device according to any one of claims 1 to 9, **characterized in that** the protective sheath (16) is arranged between the syringe body (10) and a support sheath (18).

## Patentansprüche

1. Gesicherte Injektionsvorrichtung, umfassend eine Spritze mit einem Spritzenkörper (10), einer Nadel (11) und einem Kolben (12), der geeignet ist, für eine Injektion in diesem Körper bewegt zu werden, sowie Sicherheitsmittel, die eine Schutzhülse (16) umfassen, wobei der Spritzenkörper und die Schutzhülse (16) zwischen einer Injektionsausführung, in der die Nadel (11) aus der Schutzhülse (16), welche um den Spritzenkörper (10) herum angeordnet ist, hinausragt, und einer Schutzausführung, in der die Nadel innerhalb der Hülse verläuft, zueinander verschieblich sind, wobei die Vorrichtung ein Auslöseorgan (30, 52) umfaßt, das mit dem Betätigungskopf (12B) des Kolbens (12) fest verbunden und geeignet ist, den Übergang von der Injektionsausführung in die Schutzausführung in der Injektionsendstellung zu bewirken, wobei die Vorrichtung ein Hemmorgan (34, 50) umfaßt, das geeignet ist, eine Ausgangshemmposition einzunehmen, in der das Hemmorgan (34, 50) eine erste Injektionsendstellung des Kolbens definiert, in welcher das Auslöseorgan (30, 52) nicht in der Lage ist, den Übergang von der Injektionsausführung in die Schutzausführung zu bewirken, sowie von dem Kolben (12) getrennt oder ihm gegenüber bewegt zu werden, um eine zweite Injektionsendstellung des Kolbens zu ermöglichen, in der das Auslöseorgan (30, 52) in der Lage ist, den Übergang von der Injektionsausführung in die Schutzausführung zu bewirken,
**dadurch gekennzeichnet, daß** das Hemmorgan (34, 50) in seiner Ausgangshemmposition auf den Betätigungskopf (12B) des Kolbens (12) aufgesetzt ist, so daß der Benutzer auf das Hemmorgan (34, 50) drückt, um den Kolben (12) bis zu seiner ersten Injektionsendstellung zu bewegen, und daß der Benutzer für den Übergang von der Hemmposition in die zweite Position, wobei das Hemmorgan (34, 50) gegenüber dem Kolben (12) bewegt oder hiervon getrennt wird, auf den Betätigungskopf (12B) des Kolbens drückt.

2. Vorrichtung nach Anspruch 1, **dadurch gekennzeichnet, daß** das Hemmorgan (34) in seiner Hemmposition den Kopf (12B) des Kolbens (12) durchgreift.

3. Vorrichtung nach Anspruch 2, **dadurch gekennzeichnet, daß** ein Längsabschnitt des Hemmorgans (34), der von einem Zapfen oder von einem Wandelement (38) gebildet ist, den Betätigungskopf (12B) des Kolbens durch einen Schlitz (36) durchgreift.

4. Vorrichtung nach einem der Ansprüche 1 bis 3, **dadurch gekennzeichnet, daß** sie Anschlagmittel (38A, 10B) umfaßt, die geeignet sind, in Gebrauch gesetzt zu werden, um die erste Injektionsendstellung zu definieren, und außer Gebrauch gesetzt zu werden, um das Erreichen der zweiten Injektionsendstellung zu ermöglichen.

5. Vorrichtung nach Anspruch 4, **dadurch gekennzeichnet, daß** das Hemmorgan (34) in seiner Hemmposition geeignet ist, mit einem Element (10B) der Vorrichtung, das gegenüber dem Spritzenkörper (10) fest ist, anschlagend zusammenzuwirken, um die erste Injektionsendstellung zu definieren.

6. Vorrichtung nach einem der Ansprüche 1 bis 5, **dadurch gekennzeichnet, daß** sie eine Aufnahme (40) umfaßt, in welcher der Kopf (12B) des Kolbens (12) in der zweiten Injektionsendstellung im wesentlichen eingezogen ist, und daß in der ersten Injektionsendstellung der Kopf des Kolbens aus dieser Aufnahme hinausragt, um eine Greifstelle zu bieten, die ermöglicht, den Kolben -ihn von der Nadel entfernend- zu ziehen.

7. Vorrichtung nach Anspruch 1, **dadurch gekennzeichnet, daß** das Hemmorgan eine Kappe (50) ist, von der ein Mantelteil (52) das Auslöseorgan bildet und die gegenüber dem Kolben (12) axial beweglich ist, wobei die für das Auslösen geeignete Position gegenüber der für das Auslösen ungeeigneten Position in Richtung des der Nadel zugewandten Endes (12A) des Kolbens (12) versetzt ist.

8. Vorrichtung nach Anspruch 7, **dadurch gekennzeichnet, daß** das Hemmorgan Mittel (53, 54A, 54B) umfaßt, um eine schwergängige Stelle beim Verschieben der Kappe (50) gegenüber dem Kolben (12) zu definieren, um das Auslöseorgan (52) in seiner für das Auslösen ungeeigneten Position zu halten.

9. Vorrichtung nach einem der Ansprüche 1 bis 8, **dadurch gekennzeichnet, daß** das Auslöseorgan (30, 52) von einem Mantel gebildet ist, der geeignet ist, über Laschen (26) mit elastischen Laschen (17) der Schutzhülse (16) zusammenzuwirken, um den Übergang von der Injektionsausführung in die Schutzausführung zu bewirken.

10. Vorrichtung nach einem der Ansprüche 1 bis 9, **dadurch gekennzeichnet, daß** die Schutzhülse (16) zwischen dem Spritzenkörper (10) und einer Traghülse (18) angeordnet ist.
